(19) Europäisches Patentamt
European Patent Office
Office européen des brevets

(11)  **EP 2 871 478 B1**

## (12)  EUROPEAN PATENT SPECIFICATION

(45) Date of publication and mention
of the grant of the patent:
**29.04.2020  Bulletin 2020/18**

(51) Int Cl.:
**G01N 33/28** *(2006.01)*   **G01N 23/12** *(2018.01)*

(21) Application number: **13306529.2**

(22) Date of filing: **08.11.2013**

(54)  **Method for analyzing fluid and multiphase flow meter**

Verfahren zur Analyse eines Fluids und Mehrphasenströmungsmesser

Méthode d'analyse de fluide et débitmètre multiphase

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.05.2015  Bulletin 2015/20**

(73) Proprietors:
• **Services Petroliers Schlumberger SA
75007 Paris (FR)**
Designated Contracting States:
**FR**
• **Schlumberger Technology B.V.
2514 JG The Hague (NL)**
Designated Contracting States:
**AL AT BE BG CH CY CZ DE DK EE ES FI GR HR HU IE IS IT LI LT LU LV MC MK MT NO PL PT RO RS SE SI SK SM TR**
• **Schlumberger Holdings Limited
Road Town, Tortola 1110 (VG)**
Designated Contracting States:
**GB NL**

(72) Inventors:
• **Chazal, Damien
95971 Roissy-en-France (FR)**

• **Fiore, Massimiliano
95971 Roissy-en-France (FR)**

(74) Representative: **Schlumberger Intellectual Property Department
Parkstraat 83
2514 JG Den Haag (NL)**

(56) References cited:
**EP-A1- 2 574 919     WO-A2-2009/036337**

• **LECLAIR ROBERT ET AL: "An analytic model for the response of a CZT detector in diagnostic energy dispersive x-ray spectroscopy", MEDICAL PHYSICS, AIP, MELVILLE, NY, US, vol. 33, no. 5, 24 April 2006 (2006-04-24) , pages 1329-1337, XP012092101, ISSN: 0094-2405, DOI: 10.1118/1.2190331**
• **KRUMREY M ET AL: "Calibration and characterization of semiconductor X-ray detectors with synchrotron radiation", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 568, no. 1, 30 November 2006 (2006-11-30), pages 364-368, XP027925645, ISSN: 0168-9002 [retrieved on 2006-11-30]**

**Description**

**BACKGROUND**

Wells are generally drilled into subsurface rocks to access fluids, such as hydrocarbons, stored in subterranean formations. The subterranean fluids can be produced from these wells through known techniques. Operators may want to know certain characteristics of produced fluids to facilitate efficient and economic exploration and production. For example, operators may want to know flow rates of produced fluids. These produced fluids are often multiphase fluids (e.g., those having some combination of water, oil, and gas), making measurement of the flow rates more complex.

Various systems can be used to determine flow rates for multiphase fluids. In some systems, multiphase fluids are separated into their constituent phases and these phases are then individually tested to determine flow rates. Other systems include multiphase flow meters that can be used to measure flow rates of multiphase fluids without separation. These multiphase flow meters may be smaller and lighter than traditional separators and test units, and the ability to measure flow rates without separation may be desirable in some instances. Both the traditional separator systems and the multiphase flow meter systems can also be used to determine certain other fluid characteristics of interest.

EP 2 574 919 A1 discloses an apparatus for fluid phase fraction determination, comprising an x-ray generator, a pipe section, a detector, a multichannel analyzer and a control arrangement. The detector comprises a scintillation crystal and a photomultiplier. Low and high energy radiation is transmitted through the fluid and the spectrum is detected by the detector. The multiphase fluid mixture properties are determined based on a deconvolution of the spectrum.

EP 1 970 702 A1 refers to a method of detecting an additional element from a plurality of other elements forming multiphase flow that involves measuring energy spectrum response, determining fraction concentration of other elements, and detecting an additional element.

WO 02/50522 A1 refers to an apparatus for characterizing multi-phase liquid effluent that includes scintillation crystal differentiating first and second energy levels emitted by gamma ray source.

Lavigne, et al., "Extraordinary improvement in scintillation detectors via post-processing with ASEDRA-solution to a 50-year-old problem," SPIE 1 January 2008, refers to method of processing a detector spectrum based on ASEDRA or "Advanced Synthetically Enhanced Detector Resolution Algorithm" that includes an ACHIP or "Adaptive Chi-square Processed denoising".

WO 2009/036337 A2 refers to a method of processing a detector spectrum e.g. scintillation detector spectrum that involves subtracting detector response function corresponding to identified photopeak from detector spectrum.

Meng and D Ramsden L J: "An inter-comparison of three spectral-deconvoultion algorithms for gammy-ray spectroscopy," IEEE Transactions on Nuclear Science, vol. 47, no. 4, pt 1, 1 August 2000, pages 1329-1336, refers to a comparison of three deconvolution techniques, Maximum Likelihood, Maximum Entropy, and Linear Regularization, for unconstrained deconvolution of gamma-ray spectra.

WO 01/25762 A1 refers to an apparatus for measuring the phase volume fractions of multi-phase mixture that has a conduit with reduced diameter section, a source of radiation, a detector, a section of low radiation absorption material, and a processor.

WO 2009/058964 A1 refers to a method of measuring the composition of multiphase fluid. The method includes radiating photon beam via multiphase fluid; measuring radiation absorption, and providing data to a processing unit configured to calculate composition of multiphase fluid.

LeClair Robert, et al., "An analytic model for the response of a CZT detector in diagnostic energy dispersive x-ray spectroscopy," Medical Physics, AIP, Melville, NY, US, vol. 33, no. 5, 24 April 2006, pages 1329-1337, refers to an analytical model to characterize the poor hole transport properties and fluorescence generation upon atomic de-excitations of CdZnTe detectors, which occur when exposed to diagnostic x-rays.

Krumrey, M, et al., "Calibration and characterization of semiconductor X-ray detectors with synchrotron radiation," Nuclear Instruments & Methods in Physics Research, Section A: Accelerators, Spectrometers, Detectors, and Associated Equipment, Elsevier BV, vol. 568, no. 1, 30 November 2006, pages 364-368, refers to different techniques for the calibration of energy-dispersive X-ray detectors such as detectors based on silicon, germanium, or CdTe as well as for silicon photodiodes. One technique includes a storage ring as primary source standard where the detector response to the calculable spectral photon flux is measured in the undispersed synchrotron radiation. Another technique includes the comparison to a primary detector standard, mainly a cryogenic electrical substitution radiometer.

**SUMMARY**

The present invention relates to a method according to independent claim 1 and a multiphase flow meter according to independent claim 8. Preferred embodiments are defined in the dependent claims.

## BRIEF DESCRIPTION OF THE DRAWINGS

These embodiments will become better understood when the following detailed description is read with reference to the accompanying drawings in which like characters represent like parts throughout the drawings, wherein:

FIG. 1 generally depicts an apparatus in the form of a flow meter for analyzing a fluid;

FIG. 2 is a block diagram of components of a computer of the apparatus of FIG. 1;

FIGS. 3 and 4 generally depict an emitter and a detector of electromagnetic radiation positioned about a fluid conduit to enable irradiation of fluid within the conduit and measurement of radiation transmitted through the fluid;

FIG. 5 is a block diagram of components of the emitter and detector of FIGS. 3 and 4;

FIG. 6 generally depicts a detector having a scintillation crystal, a photomultiplier tube, and an amplifier;

FIG. 7 is a flow chart for developing a response model for the detector of FIG. 6;

FIGS. 8-13 generally illustrate various interaction effects of electromagnetic energy with a scintillation crystal and impacts on the deposited spectrum;

FIG. 14 depicts electromagnetic emissions of barium-133 at various energy levels;

FIG. 15 depicts a simulated crystal response function;

FIG. 16 generally depicts determination of a crystal impulse response for a scintillation crystal;

FIG. 17 depicts simulated individual spectral responses of a scintillation crystal for various incident energy levels;

FIG. 18 generally illustrates additional details of the photomultiplier tube of FIG. 6;

FIG. 19 depicts deconvolution kernel components based on the individual spectral responses of FIG. 17;

FIG. 20 is a block diagram of electronic components of the apparatus of FIG. 1;

FIG. 21 is a graph generally depicting synchronous pulses generated by the photomultiplier tube;

FIG. 22 is a flow chart for inferring incident count rates using a physical model of a detector;

FIG. 23 is a flow chart for determining phase fractions of a fluid through spectrum deconvolution;

FIGS. 24-27 are examples of spectrum deconvolutions for various radioactive sources and detector types;

FIG. 28 is a flow chart for calculating incident count rates, attenuation, and phase fractions of a fluid; and

FIG. 29 is a flow chart for optimizing variables of a detector response model to calculate characteristics of a fluid of interest.

## DETAILED DESCRIPTION OF SPECIFIC EMBODIMENTS

It is to be understood that the present disclosure provides many different embodiments or examples for implementing different features of various embodiments. Specific examples of components and arrangements are described below for purposes of explanation and to simplify the present disclosure. These are, of course, merely examples and are not intended to be limiting.

When introducing elements of various embodiments, the articles "a," "an," "the," and "said" are intended to mean that there are one or more of the elements. The terms "comprising," "including," and "having" are intended to be inclusive and mean that there may be additional elements other than the listed elements. Moreover, any use of "top," "bottom," "above," "below," other directional terms, and variations of these terms is made for convenience, but does not mandate any particular orientation of the components.

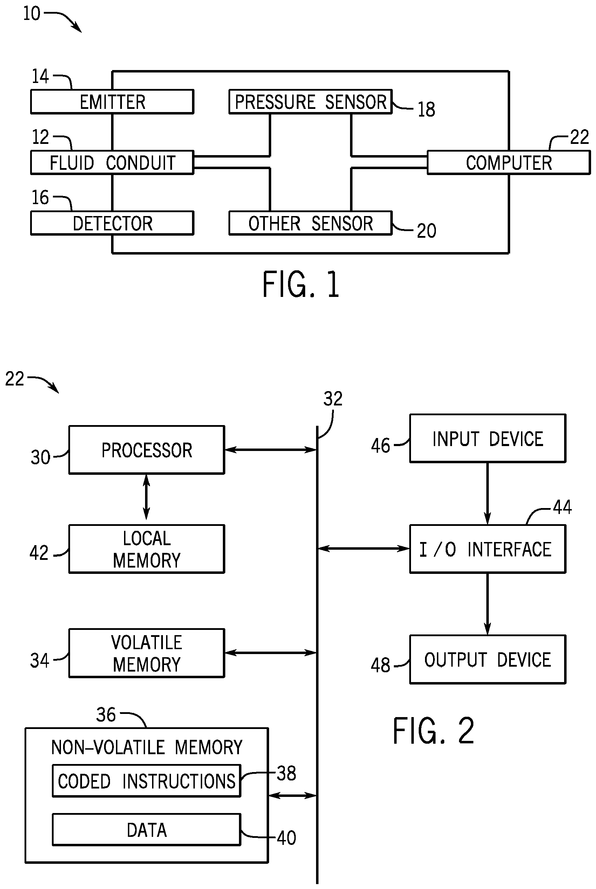

Turning now to the drawings, an apparatus 10 in the form of a flow meter is generally depicted in FIG. 1 in accordance with one embodiment. While certain elements of the apparatus 10 are depicted in this figure and generally discussed below, it will be appreciated that the apparatus 10 may include other components in addition to, or in place of, those presently illustrated and discussed. The apparatus 10 is provided in the form of a multiphase flow meter as described below.

As depicted, the apparatus includes a fluid conduit 12 for receiving a fluid. The apparatus 10 also includes an emitter 14 of electromagnetic radiation, a detector 16 of electromagnetic radiation, a pressure sensor 18 (e.g., one or both of a pressure transmitter and a differential-pressure transmitter), and one or more additional sensors 20 (e.g., a temperature sensor). To facilitate certain measurements, such as flow rate, the fluid conduit 12 can have a tapered bore (e.g., a Venturi throat) to constrict fluid flow. Further, in at least one embodiment the emitter 14 and detector 16 are positioned about a Venturi throat in the fluid conduit 12 such that the detector 16 receives radiation that has been transmitted through fluid within the Venturi throat.

The apparatus 10 further includes a computer 22 (which may also be variously referred to as a controller or a control unit) for determining characteristics of fluid within the fluid conduit 12. In at least some embodiments, the computer 22 is provided in the form of a flow computer coupled with the other depicted components in a single unit to facilitate installation of a flow meter in a larger system (e.g., an oilfield apparatus). More specifically, the computer 22 is operable to determine characteristics of fluid within the fluid conduit 12 from measurements collected by the other components. For example, the computer 22 can determine pressure and flow rate of the fluid. Further, a computer 22 of a multiphase flow meter can determine the attenuation of the fluid with respect to various levels of electromagnetic radiation by comparing the amount of radiation emitted from the emitter 14 to the portion of such radiation actually received by the detector 16. Such a computer 22 can also use this information to calculate phase fractions (e.g., proportions of oil, gas, and water) for a multiphase fluid within the fluid conduit 12. Finally, single-phase flow rates can be achieved by combining the phase fraction measurements together with the total flow rate measurement. Often, a multiphase flow model is implemented to compensate for differences between the velocities of liquid and gas in the fluid.

The computer 22 is a processor-based system, an example of which is provided in FIG. 2. In this depicted example, the computer 22 includes at least one processor 30 connected, by a bus 32, to volatile memory 34 (e.g., random-access memory) and non-volatile memory 36 (e.g., flash memory and a read-only memory (ROM)). Coded application instructions 38 and data 40 are stored in the non-volatile memory 34. For example, the application instructions 38 can be stored in a ROM and the data 40 can be stored in a flash memory. The instructions 38 and the data 40 may be also be loaded into the volatile memory 34 (or in a local memory 42 of the processor) as desired, such as to reduce latency and increase operating efficiency of the computer 22. The coded application instructions 38 can be provided as software that may be executed by the processor 30 to enable various functionalities described herein. Non-limiting examples of these functionalities include deconvolution of a measured energy spectrum, determination of incident photon count rates on a detector, and calculation of attenuation rates and phase fractions for a fluid. In at least some examples, the application instructions 38 are encoded in a non-transitory computer readable storage medium, such as the volatile memory 34, the non-volatile memory 36, the local memory 42, or a portable storage device (e.g., a flash drive or a compact disc).

An interface 44 of the computer 22 enables communication between the processor 30 and various input devices 46 and output devices 48. The interface 44 can include any suitable device that enables such communication, such as a modem or a serial port. In some examples, the input devices 46 include one or more sensing components of the apparatus 10 (e.g., detector 16, pressure sensors 18, other sensors 20) and the output devices 48 include displays, printers, and storage devices that allow output of data received or generated by the computer 22. Input devices 46 and output devices 48 may be provided as part of the computer 22 or may be separately provided.

Further, while the computer 22 could be located with the fluid conduit 12 and sensing components of the apparatus 10 as a unitary system (e.g., a flow meter), the computer 22 could also be located remote from the other components. Further, the computer 22 could be provided as a distributed system with a portion of the computer 22 located with the sensing components at the fluid conduit 12 and the remaining portion of the computer 22 located remote from the fluid conduit 12.

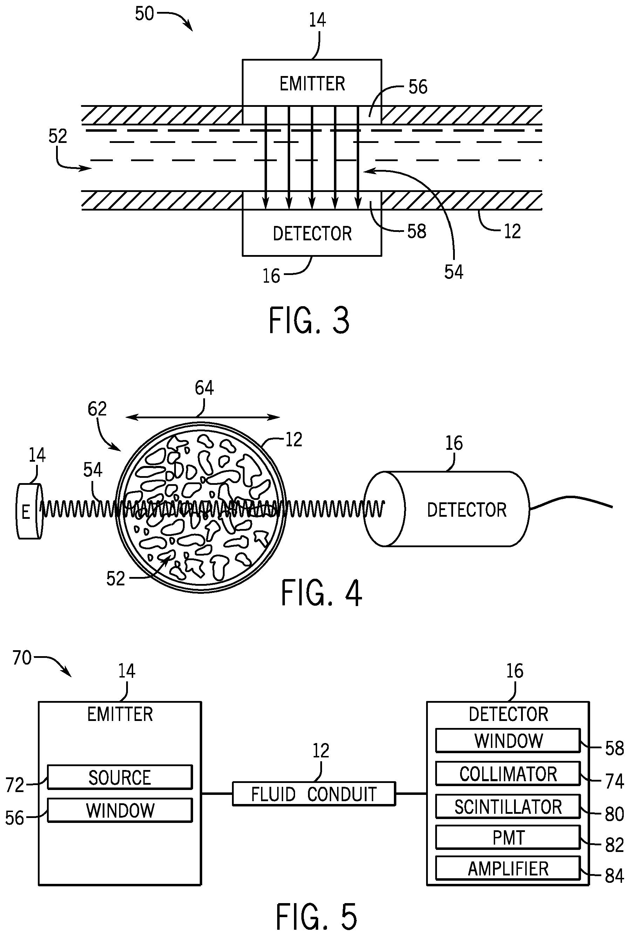

Additional details regarding operation of the emitter 14 and the detector 16 may be better understood with reference to FIGS. 3 and 4. The emitter 14 and the detector 16, which may also be referred to as components of a spectrometer or densitometer 50, are arranged about the fluid conduit 12 in any suitable manner that allows the detector 16 to receive electromagnetic radiation transmitted through fluid within the fluid conduit 12 from the emitter 14. As presently shown, the emitter 14 and the detector 16 are coupled opposite one another about the fluid conduit 12. Fluid 52 within the fluid conduit 12 is irradiated with electromagnetic radiation 54. Some of the electromagnetic radiation 54 is absorbed or scattered by the fluid 52, but a portion of the electromagnetic radiation 54 is received by the detector 16. Windows 56 and 58 isolate the emitter 14 and the detector 16 from the fluid 52, while still permitting the electromagnetic radiation 54 to be transmitted from the emitter 14 and received by the detector 16. Particularly, the windows 56 and 58 are at least partially transparent to electromagnetic radiation to be emitted from the emitter 14 and read by the detector 16.

The emitter 14 produces x-rays and gamma rays. For instance, in some embodiments the emitter 14 includes one or more radioactive sources that emit gamma rays and x-rays. Other embodiments could include non-radioactive emitters 14, such as an electric x-ray generator.

As generally shown in FIG. 4, the emitter 14 and the detector 16 can be positioned on opposite sides of a Venturi throat 62 in the fluid conduit 12. This arrangement allows measurement of the linear attenuation coefficient, $\lambda_m(E)$, of the fluid 52 for electromagnetic radiation at a given energy $E$ according to the Beer-Lambert law:

$$\lambda_m(E) = \frac{1}{d}\ln\bigl(N_0(E)/N(E)\bigr),$$

in which $d$ is the throat diameter 64, $N(E)$ is the amount of transmitted photons (the quantity of photons detected by the detector 16), and $N_0(E)$ is the empty pipe count rates (the quantity of photons emitted from the emitter 14 that would reach the detector 16 but for interference by a medium, such as the fluid 52, in the throat 62).

In some instances, the analyzed fluid can have multiple phases. For example, the fluid 52 can be a multiphase fluid having an oily liquid phase, an aqueous liquid phase, and a gaseous phase, which may be more generally referred to as oil, water, and gas phases. It will be appreciated by those skilled in the art that the attenuation of electromagnetic radiation by a multiphase fluid is a linear combination of the attenuations caused by each of its phases weighted by their proportions in the fluid. In the case of a fluid having some combination of oil, water, and gas, this can be written as:

$$\lambda_m(E) = \lambda_g(E)\,\alpha_g + \lambda_w(E)\,\alpha_w + \lambda_o(E)\,\alpha_o,$$

where $\lambda_g$, $\lambda_w$, and $\lambda_o$ are attenuation coefficients for gas, water, and oil for radiation of a given energy level $E$, and $\alpha_g$, $\alpha_w$, and $\alpha_o$ are respective fractional portions of each phase within the analyzed fluid (also referred to herein as phase hold-ups or phase fractions).

This gives as many equations as the number of distinct energy levels in the electromagnetic radiation from the emitter 14. Further considering that the three phase hold-ups sum up to 1, the following system of linear equations can be achieved:

$$\begin{pmatrix} \lambda_g(E_1) & \lambda_w(E_1) & \lambda_o(E_1) \\ \vdots & \vdots & \vdots \\ \lambda_g(E_n) & \lambda_w(E_n) & \lambda_o(E_n) \\ 1 & 1 & 1 \end{pmatrix} \cdot \begin{pmatrix} \alpha_g \\ \alpha_w \\ \alpha_o \end{pmatrix} = \begin{pmatrix} \lambda_m(E_1) \\ \vdots \\ \lambda_m(E_n) \\ 1 \end{pmatrix}$$

The attenuation matrix above (i.e., the matrix including the phase-specific attenuation coefficients for n energy levels) can be obtained from full bore measurements on each phase, hereafter called the in-situ references, or theoretical coefficients can be used. This attenuation matrix can then be inverted (giving an inversion matrix $\mathbf{A}^{-1}$) to calculate the phase hold-ups:

$$\begin{pmatrix} \alpha_g \\ \alpha_w \\ \alpha_o \end{pmatrix} = \mathbf{A}^{-1} \cdot \begin{pmatrix} \lambda_m(E_1) \\ \vdots \\ \lambda_m(E_n) \\ 1 \end{pmatrix}$$

The equations above relating the phase attenuations and phase fractions to the measured attenuation coefficients for the multiphase fluid assume the energy levels $E_1 \ldots E_n$ emitted from a source can be independently measured by the detector. In reality, however, the detector response is not ideal and some higher-energy photons can be accounted in lower-energy regions or, conversely, lower-energy photons can be accounted in higher-energy regions. Due to this mixing of incident energies, the phase hold-ups can eventually be biased when inverting the attenuation matrix. Likewise, the detector response may drift over time because of temperature fluctuations or due to its own aging. As a consequence, the real-time count rates may differ from the in-situ references (which could have been acquired several days or months before, for example). This can also lead to a systematic error on the phase hold-ups.

To compensate for these two sources of error, each part of the detection process can be modeled. Moreover, rather than recording just certain electromagnetic emissions and then using an empirical model to compensate for variance between real and ideal detector response, at least some embodiments of the present disclosure use the detector 16 to measure the full energy spectrum of the electromagnetic radiation 54. And as described in greater detail below, such embodiments then use a physical model of the response of the detector 16 to determine count rates for photons of different energy levels of interest that are incident on the detector 16. In at least some instances, the measurement of the full energy spectrum and use of the physical model enables the detection chain of the apparatus 10 to be insensitive to temperature, aging drifts, and source activity variations. These features also allow the presently disclosed techniques for determining incident count rates to be broadly applicable to any types of sources, detector technologies, and source-detector geometries.

Additional features of the emitter 14 and the detector 16 are depicted in FIG. 5 as part of a system 70 . In this example, the emitter 14 includes a source 72 of electromagnetic radiation. As noted above, the source 72 can be a radioactive source, such as barium-133 or americium-241. The selection of the source 72 can be based on the fluid intended to be analyzed. For instance, americium-241 could be used if the fluid 52 is a wet gas and barium-133 could be used in other cases. Fluorescent sources, which generally emit lower-energy spectra than radioactive sources, could also be used. In addition to the windows 56 and 58 described above, the system 70 includes a collimator 74. The collimator 74 has an opening, such as a slit, that forms a beam of electromagnetic radiation that is directed toward the scintillator 80. As presently depicted, the collimator 74 is on the detector side of the system, so that electromagnetic radiation transmitted through the fluid is collimated for receipt by the scintillator 80. This helps filter out scattered photons from the radiation passed to the scintillator 80. But the collimator 74 could be provided at other positions within the system 70.

The detector 16 is illustrated as a scintillation detector in FIG. 5, though the detector could be a solid-state detector in other examples. As depicted, the detector 16 includes a scintillator 80, a photomultiplier tube (PMT) 82, and an amplifier 84. The scintillator 80 can be provided in various forms, such as a crystal. In some examples, the scintillator 80 is a NaI(T1) (sodium iodide doped with thallium) detector or a YAP(Ce) (yttrium aluminum perovskite activated with cerium) detector.

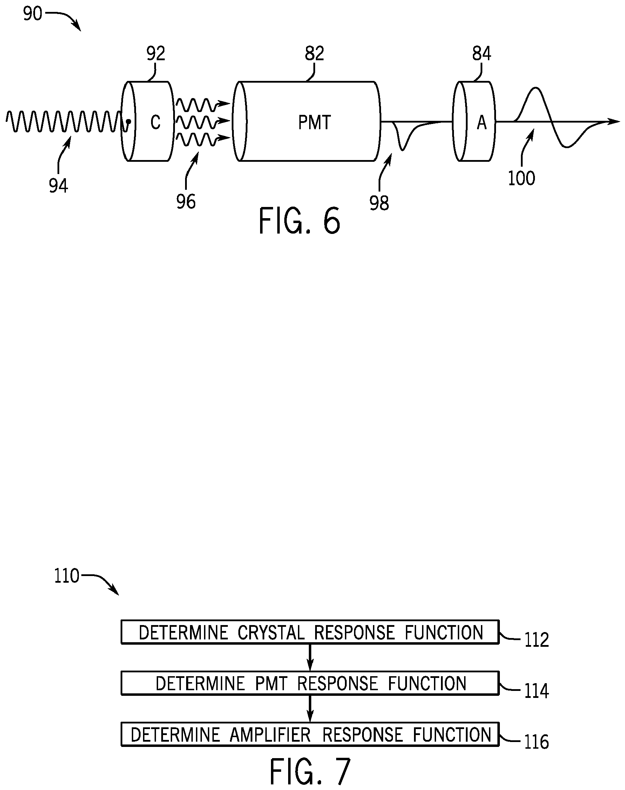

The scintillator 80 collects at least a portion of the incident photon energy it receives and converts this incident energy into radiation in a different part of the electromagnetic spectrum. For example, as depicted as part of a detection chain 90 in FIG. 6, high-energy radiation 94 (x-rays and gamma rays) can be absorbed by the scintillator (here provided as a scintillation crystal 92) to cause it to radiate pulses of light 96, such as visible light. The PMT 82, which can be optically coupled to the scintillation crystal 92, detects electromagnetic radiation (e.g., light 96) emitted from the scintillation crystal 92 and converts this radiation into electrical charges 98. The amplifier 84 then transforms these electrical charges into electrical signals, such as voltage pulses 100, suitable for analog-to-digital processing.

A physical model of the response of the detector 16 is created. The physical model can generally include models for each part of a detection chain. This physical model is stored in the computer 22 and, as described below, is used to facilitate determination of incident count rates at the detector 16 and the calculation of phase fractions for an analyzed fluid.

One example of a process for creating a physical model of the response of a scintillation detector is generally represented by flow chart 110 in FIG. 7. In this example, the components of the detection chain 90 are themselves modeled as response functions that relate inputs at each component to corresponding outputs. Particularly, as shown in FIG. 7, a response function for the scintillation crystal 92 is determined at block 112, a response function for the PMT 82 is determined at block 114, and a response function for the amplifier 84 is determined at block 116. It will be appreciated, however, that components of a solid-state detector could be similarly modeled in another example. The determination of these response functions for a scintillation detector is described in greater detail below by way of example.

In x-ray and gamma-ray spectroscopy, photons deposit their energy into a detector (e.g., the scintillation crystal 92 or a semiconductor) through matter interaction effects, thus generating an energy spectrum. Even by considering an ideally perfect deposited-energy-to-electric-signal conversion process resulting in a discrete spectrum, due to the finite size of the detector the recovered spectrum is continuous: photons emitted with energy hv have a probability of being measured with smaller energies. As described in detail below, the present invention includes inferring the number and energy of photons incident on the detector from a measured spectrum. It should be appreciated that the accuracy of such inferences will depend on the accuracy of a physical model of the detector response.

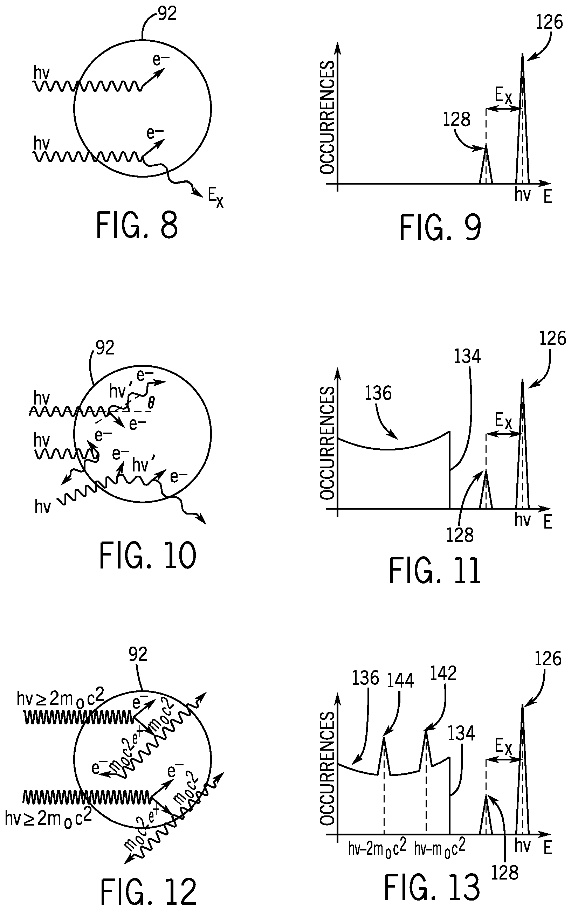

Determining the crystal response function at block 112 includes determining a crystal impulse response $h(e',e)$ that relates an incident spectrum $i(e)$ of the electromagnetic radiation 94 on the scintillation crystal 92 to a deposited spectrum $d(e)$ inside the scintillation crystal. As will be appreciated, photons in the electromagnetic radiation 94 interact with atoms of the scintillation crystal 92 to generate light 96. Examples of such interactions are generally described below with reference to FIGS. 8-13. For the sake of simplicity, these examples depict photons with energy hv hitting a scintillation crystal 92 of finite size. Notable mechanisms of gamma-ray and x-ray interaction with matter include photoelectric absorption, Compton scattering (incoherent scattering), and, in the case of gamma-rays with energy hν > 1.022 MeV, pair production.

In the case of photoelectric absorption generally depicted in FIG. 8, the incident gamma-ray (or x-ray) interacts with an electron of an atom of the scintillation crystal 92 (e.g., an electron of the inner electron shell (K-shell) of the atom) and disappears by giving up its energy hν. An electron (e⁻) is produced from this interaction (i.e., ejected from the atom receiving the incident gamma-ray or x-ray) along with either an x-ray photon or a so-called Auger electron following electron rearrangement due to the vacancy left by the ejected electron. For atomic numbers Z > 39, the probability of generating an x-ray photon is greater than seventy percent and increases with Z. The incident photon energy is often fully deposited in the detector (as is the case with the upper incident ray in FIG. 8), thus contributing to a full-energy peak 126 as depicted in FIG. 9. However, if the effect takes place near the detector surface, an x-ray photon of energy $E_x$ may leave the detector (as is the case with the lower incident ray in FIG. 8). The deposited energy will then be $hν - E_x$, corresponding to a characteristic x-ray escape peak (EP) 128 in the spectrum shown in FIG. 9.

As generally shown in FIG. 10, in the case of Compton scattering the incident gamma-ray (or x-ray) with energy hν interacts with an electron by giving up part of its energy to the electron itself and being scattered at an angle $\theta$. The portion of energy between the recoil electron and the scattered photon of energy $hν' \leq hν$ depends on the scattering angle $\theta$. When both Compton scattering products deposit their energy in the detector (when the scattered photon is eventually photoelectric-absorbed as is the case with the uppermost incident ray in FIG. 10), the incident photon contributes to the full-energy peak 126 depicted in FIG. 11. But when the scattered photon leaves the detector (see, e.g., the middle incident ray in FIG. 10), just the recoil electron energy is deposited. The maximum recoil electron energy corresponds to a head-on collision, i.e., $\theta = \pi$, and is given by:

$$ E_{e^-,\theta=\pi} = hν \frac{2hν/m_0c^2}{1 + 2hν/m_0c^2} $$

In the spectrum of FIG. 11, this is represented by the Compton edge (CE) 134 at energy $E_{e^-,\theta=\pi}$ and, for $0 \leq \theta \leq \pi$, recoil electrons generate to the so-called Compton continuum 136. Moreover, if a scattered photon escapes the detector after multiple Compton scatterings (see, e.g., the lowermost incident ray in FIG. 10), the deposited energy will be $\varepsilon < E_{e^-} < hν - \varepsilon$, with $\varepsilon \cong 0$, thus leading to an extra background overlapping the Compton continuum for energies less than or equal to $E_{e^-,\theta=\pi}$ (which, for the sake of clarity, is not shown in FIG. 11).

Unlike the previous interaction effects, pair production is a threshold effect. In such an interaction (two of which are generally depicted in FIG. 12) a gamma-ray with energy hν disappears to create an electron-positron pair. Since the electron (and positron) rest mass $m_0$ corresponds to an energy of $m_0c^2$ = 511 keV, the pair creation can take place just if $hν \geq 2 m_0c^2$. In some instances, the annihilation photons from positron-electron annihilation (e⁺ are highly unstable particles) deposit their whole energies in the detector, thus contributing to the full-energy peak 126 in FIG. 13. In other instances, however, either one or both photons can leave the detector. This leads to the formation of single and double escape peaks 142 and 144 located in the spectrum at $hν - m_0c^2$ and $hν - 2 m_0c^2$, respectively.

Escape peak intensities and Compton continuum shape may be empirically determined for emissions at a particular energy level with a given detector size and hardware geometry. In the more general scenario of several gamma-ray or x-ray emissions of different energies to be detected, however, such empirical determination becomes increasingly difficult. Even an analytical approach may not provide desired results. For example, on determining the Compton continuum, the Klein-Nishina equation does not provide an accurate quantitative description of the continuum because, among other things, its free electron hypothesis is unrealistic.

In some examples, the complex problem of incident photon recovery from an energy spectrum is represented by Monte Carlo codes, where a combination of nuclear physics and statistics allows an accurate description of radiation-matter interactions. The Monte Carlo N-Particle (MCNP) transport code, developed by and available from Los Alamos National Laboratory of the United States, is used in at least some examples to simulate the response of the scintillation crystal 92 to electromagnetic radiation of different energies, although other codes or algorithms could be used for this simulation in different examples (e.g., Geant 4 from the European Organization for Nuclear Research (CERN)). Once characteristics of the three-dimensional hardware geometry, the radiation source, and the detector are introduced, MCNP takes into account detector-related effects and also simulates gamma-ray interactions with materials surrounding the detector itself. The result is a description of an ideal crystal response function (CRF).

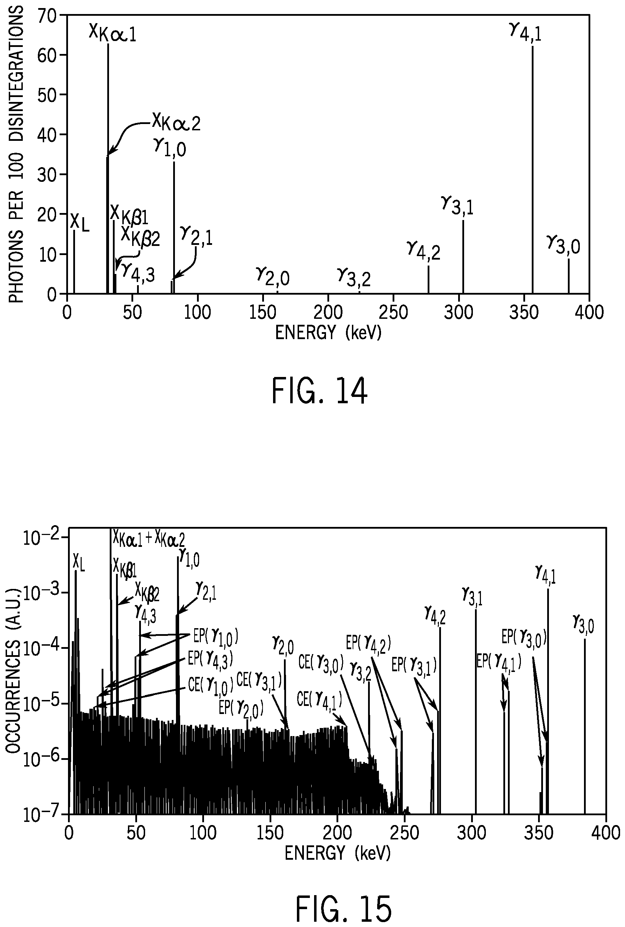

By way of example, FIG. 14 shows an x-ray and gamma-ray emission spectrum of barium-133 and FIG. 15 illustrates an ideal CRF of a 7-mm thick NaI(T1) scintillation crystal to the barium-133 radiation as simulated by MCNP. Spectral components in both of these figures are labeled with their respective generation mechanism. Although the spectrum shown in FIG. 15 is single- and double-escape peak free (as the most energetic emission from barium-133 occurs at 383.8 keV, which is less than $2 m_0c^2$), the cumulative interactive effects due to the finite detector size make the spectrum complicated, with many high energy photons sorted in low energy bins.

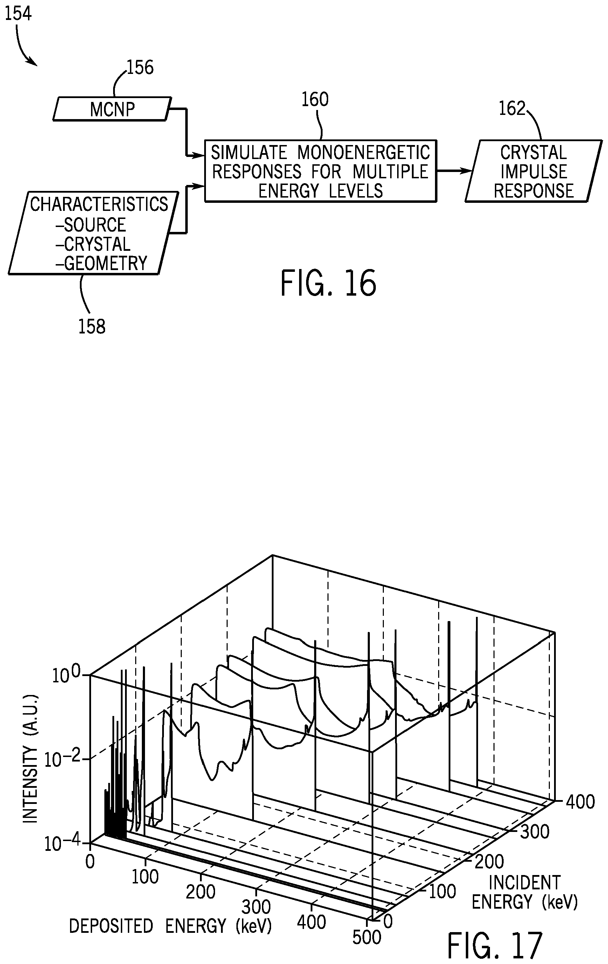

One example for modeling the impulse response of a scintillation crystal is generally depicted by flow chart 154 of FIG. 16. In order to single out the spectral contribution of each barium-133 emission, the CRF is simulated for one emission at a time. More specifically, using MCNP 156 and various characteristics 158 of the apparatus (such as characteristics of the radiation source, the detector, and the three-dimensional hardware geometry, as discussed above), monoenergetic responses for multiple energy levels are simulated (block 160) to generate a set of monoenergetic CRFs that characterize the crystal impulse response 162. The simulation of the monoenergetic responses can be performed with MCNP or in any other suitable manner. Moreover, as detailed below, this set of monoenergetic CRFs facilitates later determination of the detector incident photons from a measured spectrum.

As the energy deposition process is an energy-varying linear system, the crystal response function is fully characterized by its impulse response $h(e',e)$. Consequently, the deposited spectrum can be computed from the convolution product of the incident spectrum with $h(e',e)$:

$$d(e) = \int i(e')h(e',e)de'$$

This convolution product can also be expressed in a matrix form. If **H** denotes the energy deposition matrix into the scintillation crystal, $H_{ij} = h(e_i, e_j)$, obtained from MCNP (or other) simulations; $I$ refers to the incident spectrum vector, $I_k = i(e_k)$; and $D$ denotes the deposited spectrum vector, $D_k = d(e_k)$, then the convolution equation can also be written in discretized form as:

$$D = \mathbf{H} \cdot I$$

The crystal response matrix **H** contains the individual responses to source emissions, such as from barium-133 as discussed above. These individual responses of **H** (for the example of barium-133 source emissions) are generally depicted in FIG. 17. Particularly, FIG. 17 depicts the simulated spectral responses of the crystal for various energy levels of incident electromagnetic radiation corresponding to the gamma-ray and K x-ray spectral components of FIG. 14. In the present example, these spectral responses are for ten incident energy levels of interest (rounded to the nearest keV): 31 keV, 35 keV, 53 keV, 81 keV, 161 keV, 223 keV, 276 keV, 303 keV, 356 keV, and 384 keV. In other instances, however, the incident energy levels for which the response is simulated could differ from the preceding example. Further, monoenergetic responses could be simulated for a greater or lesser number of incident energy levels in accordance with the present techniques.

Determining the PMT response function at block 114 of FIG. 7 includes determining a PMT response function $g(e',e)$ that relates the deposited spectrum $d(e)$ to a smeared spectrum $s(e)$. While here described as a PMT response function for explanatory purposes, it is noted that a solid-state detector that does not have a PMT could also be similarly modeled. In the case of solid-state detectors, the smearing effect would result from the charge collection process rather than the electron multiplication process noted below.

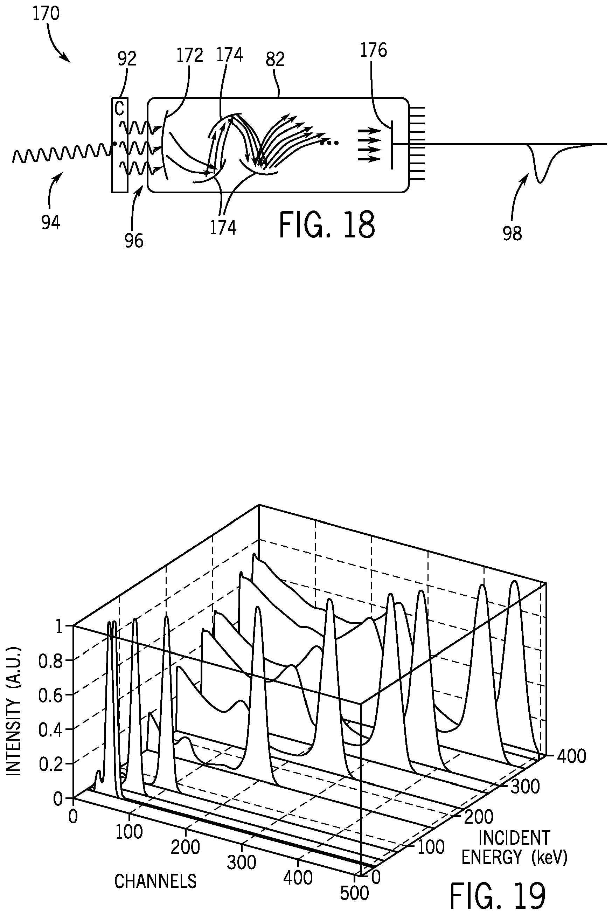

Additional details of a PMT 82 are generally illustrated in diagram 170 of FIG. 18. As previously noted, the scintillation crystal 92 converts incident radiation 94 into pulses of light 96, which is measured and converted into electrical signals 98 by the PMT 82. As depicted in the present figure, photons of light 96 from the scintillation crystal 92 fall on a light-sensitive layer in the form of a photocathode 172, causing the photocathode to emit photoelectrons. These photoelectrons are focused electrostatically onto a series of dynodes 174 that progressively amplify the current associated with the emitted photoelectrons. The amplified signal is collected at an anode 176 in the form of current pulses 98 (which can be passed to the amplifier 84 as described above).

Due to the statistical nature of the electron multiplication process of PMTs, the output charge can vary from one event to the other. This uncertainty follows a Poisson process which results in a spectral broadening (i.e., smearing) of the ideal CRF Dirac peaks. This spectral broadening can be approximated by a Gaussian filter whose parameters will depend on the crystal-PMT linearity and resolution. A photon depositing in the crystal the energy $e_j$ will be recorded in average at the channel $\mu(e_j)$ with a standard deviation $\sigma(e_j)$. These two functions (which are energy and resolution response models) are specific to each detector assembly (more specifically, the crystal-PMT assembly in those examples using a crystal scintillator) and can be parameterized the following way or based on any other energy relationship:

$$\mu(e_j) = p(1)e_j + p(2)$$

$$\sigma(e_j) = p(3)\sqrt{e_j} + p(4)$$

In some examples, the parameters $p(1)$, $p(2)$, $p(3)$, and $p(4)$ are adjusted continuously in real time to account for temperature or aging drifts that may occur over a detector's lifetime. In other examples, these parameters are adjusted continually, such as periodically at any specified frequency (e.g., once per minute).

As it is an energy-varying linear system, the PMT response function is fully characterized by its impulse response $g(e',e)$. The smeared spectrum can therefore be computed from the convolution product of the deposited spectrum with $g(e',e)$:

$$s(e) = \int d(e')g(e',e)de'$$

This convolution product can also be expressed in a matrix form. For example, if $\mathbf{G}(P)$ is a Gaussian matrix, $g_{ij} = g(e_i, e_j)$, based on the energy and resolution response models above; $D$ is the deposited spectrum vector, $D_k = d(e_k)$; and $S$ is the smeared spectrum vector, $S_k = s(e_k)$, then the convolution equation can be written in discretized form as:

$$S = \mathbf{G}(P) \cdot D$$

The product matrix $\mathbf{G(P)H}$ can be referred to as the deconvolution kernel. This deconvolution kernel characterizes the impulse response of the scintillation crystal 92 and the PMT 82. The deconvolution kernel also contains the single energy spectra (components) from which observed spectrums are created. These single energy spectra are generally depicted in FIG. 19, and it is noted that each depicted spectrum is a smeared version of an associated spectrum depicted in FIG. 17.

Returning again briefly to FIG. 7, determining the amplifier response function at block 116 includes determining an amplifier response function $f$ which relates the smeared spectrum $s(e)$ to an observed spectrum $o(e)$. Each output of the PMT 82 described above is, in essence, an amount of electrical charge proportional to the amount of energy in a photon (e.g., a gamma-ray or x-ray photon) deposited in the scintillation crystal 92. Electrical components, such as the amplifier 84 and a multi-channel analyzer, then collect that charge, measure its amplitude, and store it in the spectrum.

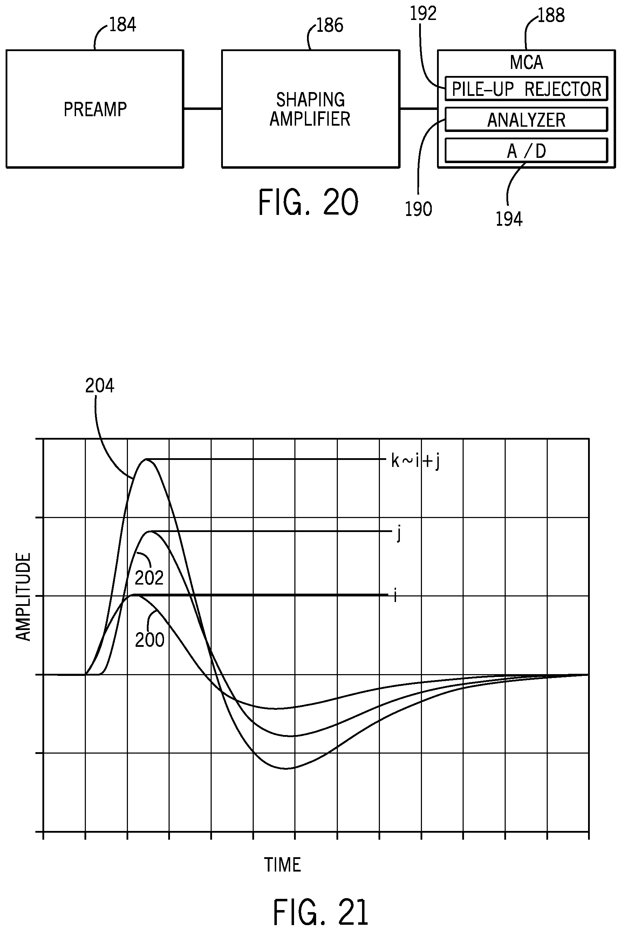

One example of such components is shown in FIG. 20. In this example, the electrical components include a preamplifier 184, a shaping amplifier 186, and a multi-channel analyzer (MCA) 188. The preamplifier 184 and the shaping amplifier 186 maybe components of the amplifier 84 (FIG. 6), while the MCA 188 can be included as part of the detector 16, part of the computer 22 (e.g., as an input device 46), or as a separate component. The preamplifier 184 converts and amplifies the current pulses 98 it receives from the PMT 82 into voltage pulses. The shaping amplifier 186 transforms these voltage pulses into linear pulses, such as unipolar or bipolar semi-Gaussian pulses, having faster baseline restoration and better signal-to-noise ratio.

The multi-channel analyzer 188 includes analyzer circuitry 190 for sorting the linear pulses into respective channels. The MCA 188 could include any suitable number of measurement channels for sorting linear pulses received from the shaping amplifier 186. For example, in some examples the MCA 188 has 512 channels or 1024 channels. When two incident photons arrive at the detector within the width of the shaping amplifier output pulse, their respective pulses pile up to form an output pulse of distorted height, leading to a distorted energy spectrum. While post-processing algorithms may be able to describe the effect of pulse pile-up on the spectrum in some instances, they can also be too resource-intensive (e.g., in CPU processing cycles) for certain (e.g., real-time) implementations.

Accordingly, the depicted MCA 188 includes a pile-up rejector 192. This pile-up rejector 192 discards pile-up events in which their time interval is longer than a threshold pile-up rejection time. The threshold pile-up rejection time can be set to any desired level, such as a level that would result in the discarding of most pile-up events. This can simplify the interpretation of the spectrum distortion by generally reducing pile-up effects to the case of synchronous pulses. In order to model the distortion due to these left-over synchronous pulses, a quantitative analysis is performed on each channel $k$ of the spectrum. It is possible to demonstrate from Poisson's law that the probability of two photons piling-up is:

$$P_0 = n_{tot}\tau \exp(-n_{tot}\tau),$$

where $\tau$ is the pile-up rejection time and $n_{tot}$ is the total count rate.

An example of synchronous pulses is generally represented in the graph of FIG. 21, in which a pulse 200 (with amplitude $i$) is synchronous with a pulse 202 (with amplitude $j$), resulting in a cumulative pulse 204 that would be read into a channel *k* due to the summation of *i* and *j*. Further, if *i* and *j* denote the incident amplitude combinations, the gains and losses rates can be calculated for each channel *k* via the following formula:

$$G_k = n_{tot} \sum_{i=1}^{k-1} P_i P_{k-i} P_0 \qquad \text{with} \quad P_k = \frac{S_k}{n_{tot}}$$
$$L_k = 2n_{tot} \sum_{i=1}^{n_{tot}} P_i P_k P_0$$

The observed spectrum is then a balance of gains and losses in each channel:

$$O_k = \mathrm{f}(S_k) = S_k - L_k + G_k$$

Each part of the detection chain has been modeled above in the form of individual response functions. The global detector response can be considered a physical model that combines those individual response functions to represent the functioning of the modeled detector chain. Consequently, this global detector response can be expressed in matrix form as:

$$O = \mathrm{f}\big(\mathbf{G}(\mathbf{P})\,\mathbf{H}\;\mathbf{I}\big)$$

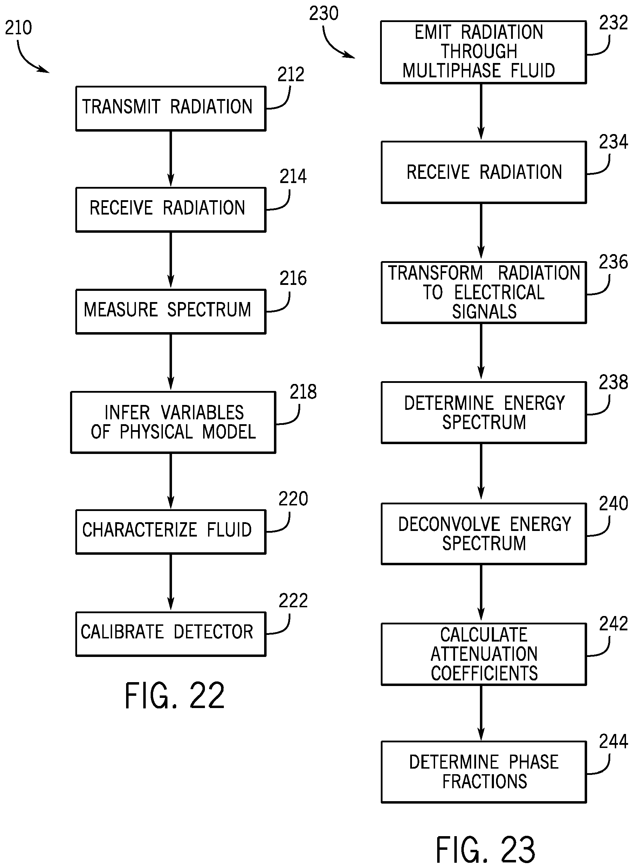

This physical model for detector response is used to infer incident count rates for photons of different energy levels received by the detector 16. The inferred count rates can then be used to determine characteristics of an analyzed fluid. One example of a process for inferring the incident count rates and then characterizing a fluid based on the inferred count rates is generally represented by flow chart 210 in FIG. 22. In this embodiment, electromagnetic radiation (x-rays and gamma rays from emitter 14) is transmitted (block 212) through a fluid of interest. The fluid attenuates the radiation such that a portion of the radiation is received (block 214) at a detector (e.g., detector 16). At block 216, the energy spectrum of the radiation received by the detector is measured, which may be performed by a multi-channel analyzer such as that described above. In at least some instances, the full energy spectrum of the received radiation is measured. In others, a partial energy spectrum can be measured, such as a portion of the energy spectrum falling within a contiguous range of multiple channels of a multi-channel analyzer. But it is noted that, as used herein, measurement of the energy spectrum (whether a full energy spectrum or a partial energy spectrum) means measurement of counts within numerous channels of a multi-channel analyzer, rather than merely measuring counts in a handful of channels (e.g., two to ten channels isolated from one another) corresponding to particular energy levels of interest. This measurement of the spectrum, rather than of a small number of individual channels, allows incident count rates to be inferred from the measured energy spectrum in accordance with the present techniques.

At block 218, the measured energy spectrum and the physical model for detector response are used to infer variables of the physical model. For the model described above, inputs of the model include crystal monoenergetic responses **H** and the detector energy and resolution functions $\mu(e)$ and $\sigma(e)$, and the variables include the incident count rates for photons of different energy levels and the detector-specific parameters p(1), p(2), p(3), and p(4). These variables are inferred through a deconvolution process based on the detector response function O.

More specifically, the detector response function of the physical model is compared to the measured energy spectrum, which includes performing optimization (e.g., least squares optimization) on the detector response function to fit the detector response function to the measured energy spectrum and infer the incident count rates and detector-specific parameters. For example, letting *Y* be the measured spectrum, a non-linear least squares algorithm can be used to determine the detector-specific parameters P and the incident count rates I that minimize the following residuals:

$$\left\| \frac{Y - O}{\sigma_Y} \right\|^2 \qquad \Leftrightarrow \qquad \left\| \frac{Y - \mathrm{f}\big(\mathbf{G}(\mathbf{P})\,\mathbf{H}\;\mathbf{I}\big)}{\sqrt{Y}} \right\|^2$$

The residuals can be weighted by the standard deviation of the measurements, i.e., the square root of counts in this case since counting processes are ordinarily assumed to follow Poisson statistics. A Levenberg-Marquardt algorithm can be used to perform the optimization (in the form of least squares minimization) or a simpler Gauss-Newton technique can be used. Still further, maximum likelihood or maximum entropy methods can be used to perform the optimization, as can any other suitable methods.

Once the incident count rates *I* are inferred, these count rates can be compared with empty pipe count rates to determine the attenuation of electromagnetic radiation by the analyzed fluid for multiple energy levels, as described above with respect to FIG. 4. The determined attenuation can then be used to characterize the fluid (block 220), such as by determining phase fractions for the fluid or information about some additional component, such as hydrogen sulfide or salts in the fluid, as discussed below. Further, the inferred detector-specific parameters P can be used to calibrate the detector (block 222), such as to maintain the spectral output of the detector at a reference position.

In accordance with another embodiment, a process for determining phase fractions of a multiphase fluid is generally represented by flow chart 230 in FIG. 23. In this embodiment, electromagnetic radiation is emitted (block 232) through a multiphase fluid. For instance, a radioactive source emits x-rays and gamma rays into a multiphase fluid flowing through a fluid conduit. The radiation incident on a detector is received (block 234) and transformed (block 236) into electrical signals as described above. It will be appreciated that the electrical signals are representative of the incident radiation.

An energy spectrum is determined (block 238) from the electrical signals and then deconvolved (block 240) to estimate quantities of photons of multiple energy levels received by the detector. The deconvolution of the determined energy spectrum (which in at least some instances is the full energy spectrum of the received radiation) is performed in the manner described above. Attenuation coefficients for the fluid can be calculated (block 242) and phase fractions can be determined (block 244) based on the attenuation coefficients as described elsewhere herein. The phase fractions in some embodiments include gas, water, and oil phases. Further, the phase fractions could include other components in addition to (or in place of) gas, water, and oil. Still further, information about additional components, such as hydrogen sulfide or salts, could also be determined, as described below.

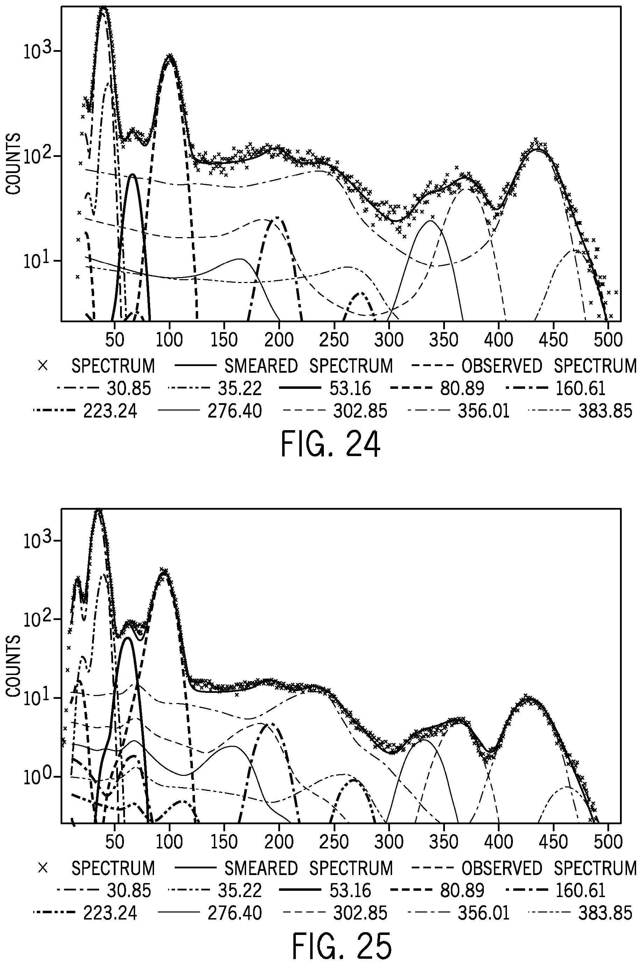

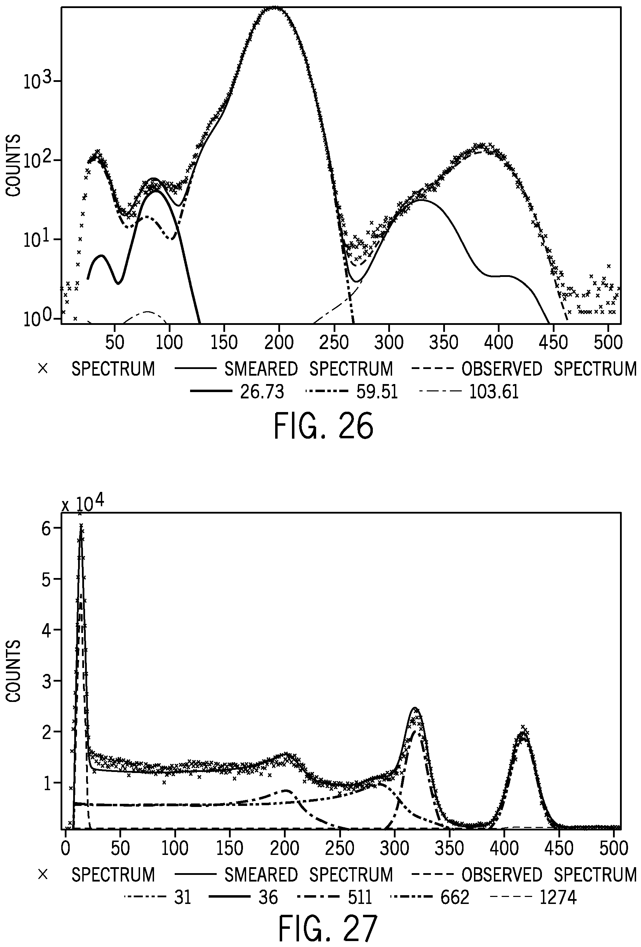

Examples of spectrum deconvolutions for various radioactive sources and detector types are generally depicted in FIGS. 24-27. In each of these examples, the deconvolution kernel is computed from MCNP simulations based on input characteristics of the radioactive source, the detector, and source-detector geometry. Further, each of these graphs depicts incident photon counts (y-axis) over 512 channels (x-axis). In addition to a measured spectrum, an observed spectrum, and a smeared spectrum, individual spectral components associated with energy lines of the respective radioactive source are also depicted (with the source energy lines enumerated in keV in at the bottom of each of these figures). The data depicted in FIG. 24 is based on a barium-133 radiation source and a 10 mm YAP(Ce) scintillation crystal. In FIG. 25, the depicted data is also based on a barium-133 radiation source, but with a 2 mm YAP(Ce) scintillation crystal. In FIG. 26, the depicted data is based on an americium-241 radiation source and a 10 mm YAP(Ce) scintillator. And the data in FIG. 27 is based on radiation source having cesium-137 and sodium-22 with a 25.4 mm (one-inch) YAP(Ce) scintillation crystal.

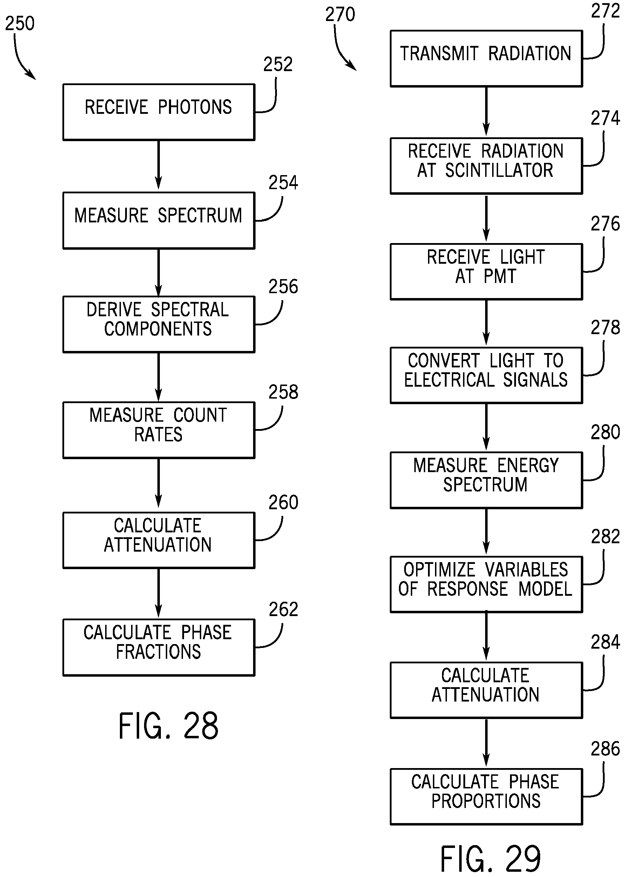

A further example of a process for calculating incident count rates, attenuation, and phase fractions of a fluid is generally represented by flow chart 250 in FIG. 28. In this embodiment, photons of different energies that have passed through a fluid of interest (e.g., a multiphase fluid in a conduit) are received at a detector (block 252) and the energy spectrum of the received photons is then measured (block 254). Spectral components of the measured energy spectrum are derived (block 256) for multiple energy levels of the photons received by the detector. These spectral components are derived by using multiple monoenergetic response functions in the manner described above. Count rates are then measured (block 258) for at least two energy levels of the received photons based on the derived spectral components. The received photons include x-ray photons and gamma-ray photons, and the at least two energy levels include a first energy level for received x-ray photons and a second energy level for received gamma-ray photons. In some embodiments, such as those using a barium-133 source, the first energy level of received x-ray photons is between 30 keV and 36 keV and the second energy level of received gamma-ray photons is between 79 keV and 81 keV. Attenuation rates of the photons by the fluid for the at least two energy levels and phase fractions for the fluid are then calculated at blocks 260 and 262 in any suitable manner.

Additionally, an example of a process that optimizes variables of a detector response model to enable calculation of fluid characteristics is generally represented by flow chart 270 in FIG. 29. In this embodiment, electromagnetic radiation is transmitted through a fluid (block 272) and an attenuated portion of that radiation is received at a scintillation crystal of a detector (block 274). The scintillation crystal emits light in response to the received radiation, and this light is received by a photomultiplier tube (block 276). The light is converted to electrical signals (block 278) to enable measurement of the energy spectrum generated by the radiation received at the scintillation crystal (block 280). Variables of a detector response model are then optimized, as described above, to minimize residuals between the measured energy spectrum and an output of the detector response model (block 282). The optimized variables of the detector response model include incident count rates for different energy levels of photons received by the scintillation detector and detector-specific parameters, as also described above. In some embodiments, the residuals are weighted based on a standard deviation of the measured energy spectrum and the variables are optimized with a non-linear, least squares algorithm, such as a Levenberg-Marquardt algorithm or a Gauss-Newton algorithm. Further, attenuation coefficients of the fluid for the different energy levels of radiation and phase proportions (e.g., for water, oil, and gas) can be calculated (blocks 284 and 286).

While the present techniques can be used to determine fractional portions for a multiphase fluid having three components (e.g., oil, water, and gas), they can also be used to determine additional components of the multiphase fluid. In some instances, the present techniques can be applied to cases of fluids having a combination of hydrocarbon liquid (e.g., oil), water, hydrocarbon gas, and some additional component, e.g., hydrogen sulfide or salts. With the present techniques, count rates from distinct energy levels in the electromagnetic radiation from the emitter 14 can be inferred. This can give as many equations as the number of the energy levels, thus providing a system of linear equations that can be inverted to calculate the fractional components of oil, water and gas as well as further information related to additional components, for instance in the form of change of salt and hydrogen sulfide quantity. The equations regarding the additional components will involve count rates of extra energy levels as well as other physical quantities depending on the chemical behavior of the additional components with oil, water and gas.

**Claims**

1. A method comprising:

   emitting x-ray and gamma radiation (54) into a fluid (52);
   receiving photons of the x-ray and gamma radiation transmitted through the fluid having different energies at a detector (16);
   physically modelling the detector by:

   determining a set of monoenergetic crystal response functions based on detector characteristics;
   determining a detector response function based on the set of monoenergetic crystal response functions, and,

   analyzing the fluid based on the photons received at the detector, wherein analyzing the fluid includes:

   measuring an energy spectrum of the photons;
   inferring incident count rates by fitting a modelled spectrum to the measured spectrum, the modelled spectrum being the detector response function applied to an incident spectrum.

2. The method of claim 1, wherein receiving the photons at the detector includes receiving photons that have passed through a multiphase fluid in a conduit (12).

3. The method of claim 2, comprising:

   calculating attenuation rates of the photons by the multiphase fluid for the at least two energy levels; and
   calculating phase fractions of the multiphase fluid using the calculated attenuation rates.

4. The method of claim 1, wherein inferring incident count rates includes inferring incident count rates for a first energy level of received x-ray photons and a second energy level of received gamma-ray photons.

5. The method of claim 4, wherein the first energy level of received x-ray photons is between 30 keV and 36 keV.

6. The method of claim 4, wherein the second energy level of received gamma-ray photons is between 79 keV and 81 keV.

7. The method of claim 1, wherein receiving the photons at the detector includes receiving the photons at a scintillator (80) of the detector.

8. A multiphase flow meter comprising:

   a fluid conduit (12);
   an emitter (14) and a detector (16) of electromagnetic radiation (54) including x- rays and gamma rays, the emitter and the detector arranged with respect to the fluid conduit so as to enable the detector (16) to receive photons transmitted from the emitter through a fluid (52) within the fluid conduit, wherein the detector includes a detection chain (90) having a scintillator (80), a photomultiplier tube (82), and an amplifier (84);
a multi-channel analyzer (188) coupled to the detector (16) to receive electrical signals from the amplifier and output a measured energy spectrum of the photons received by the detector (16); and
a flow computer (22) configured to physically model the detector by:

determining a set of monoenergetic crystal response functions based on detector characteristics, and,
determining a detector response function based on the set of monoenergetic crystal response functions, wherein the flow computer is configured to analyze the fluid, such analysis including inferring incident count rates by fitting a modelled spectrum to the measured spectrum, the modelled spectrum being the detector response function applied to an incident spectrum.

9. The multiphase flow meter of claim 8, wherein the flow computer is configured to infer the count rates for the photons received by the detector in real time.

10. The multiphase flow meter of claim 8, wherein the flow computer is configured to calculate phase fractions for the fluid within the fluid conduit in real time.

11. The multiphase flow meter of claim 8, comprising a pressure sensor (18), wherein the flow computer is configured to determine a flow rate for the fluid.

12. The multiphase flow meter of claim 8, wherein the emitter includes a radioactive source (72) of electromagnetic radiation.

13. The multiphase flow meter of claim 8, wherein the emitter includes an electric x-ray generator.

**Patentansprüche**

1. Verfahren, das umfasst:

Emittieren von Röntgen- und Gammastrahlung (54) in ein Fluid (52);
Empfangen von verschiedene Energien aufweisenden Photonen der durch das Fluid hindurchgesandten Röntgen- und Gammastrahlung an einem Detektor (16);
physikalisches Modellieren des Detektors durch:

Bestimmen eines Satzes von monoenergetischen Kristallantwortfunktionen basierend auf Detektorcharakteristika;
Bestimmen einer Detektorantwortfunktion basierend auf dem Satz von monoenergetischen Kristallantwortfunktionen, und
Analysieren des Fluids basierend auf den am Detektor empfangenen Photonen, wobei das Analysieren des Fluids umfasst:

Messen eines Energiespektrums der Photonen;
Ableiten von Einfallzählraten durch Fitten eines modellierten Spektrums an das gemessene Spektrum, wobei das modellierte Spektrum die auf ein Einfallsspektrum angewandte Detektorantwortfunktion ist.

2. Verfahren nach Anspruch 1, wobei das Empfangen der Photonen am Detektor ein Empfangen von Photonen umfasst, die durch ein Mehrphasenfluid in einer Leitung (12) hindurchgetreten sind.

3. Verfahren nach Anspruch 2, das umfasst:

Berechnen von Dämpfungsraten der Photonen durch das Mehrphasenfluid für die wenigstens zwei Energieniveaus; und
Berechnen von Phasenanteilen des Mehrphasenfluids unter Verwendung der berechneten Dämpfungsraten.

4. Verfahren nach Anspruch 1, wobei das Ableiten von Einfallzählraten ein Ableiten von Einfallzählraten für ein erstes Energieniveau von empfangenen Röntgenstrahlenphotonen und ein zweites Energieniveau von empfangenen Gammastrahlenphotonen umfasst.

**5.** Verfahren nach Anspruch 4, wobei das erste Energieniveau von empfangenen Röntgenstrahlenphotonen zwischen 30 keV und 36 keV liegt.

**6.** Verfahren nach Anspruch 4, wobei das zweite Energieniveau von empfangenen Gammastrahlenphotonen zwischen 79 keV und 81 keV liegt.

**7.** Verfahren nach Anspruch 1, wobei das Empfangen der Photonen am Detektor ein Empfangen der Photonen an einem Szintillator (80) des Detektors umfasst.

**8.** Mehrphasen-Durchflussmesser, der umfasst:

eine Fluidleitung (12);
einen Emitter (14) und einen Detektor (16) von Röntgenstrahlen und Gammastrahlen umfassender elektromagnetischer Strahlung (54), wobei der Emitter und der Detektor in Bezug auf die Fluidleitung dahingehend angeordnet sind, es dem Detektor (16) zu ermöglichen, vom Emitter durch ein Fluid (52) in der Fluidleitung hindurchgesandte Photonen zu empfangen, wobei der Detektor eine Detektionskette (90) umfasst, die einen Szintillator (80), eine Photovervielfacherröhre (82) und einen Verstärker (84) umfasst;
einen Mehrkanalanalysator (188), der mit dem Detektor (16) gekoppelt ist, um elektrische Signale aus dem Verstärker zu empfangen und ein gemessenes Energiespektrum der vom Detektor (16) empfangenen Photonen auszugeben; und
einen Durchflussrechner (22), der ausgelegt ist, den Detektor physikalisch zu modellieren, durch:

Bestimmen eines Satzes von monoenergetischen Kristallantwortfunktionen basierend auf Detektorcharakteristika, und
Bestimmen einer Detektorantwortfunktion basierend auf dem Satz von monoenergetischen Kristallantwortfunktionen, wobei der Durchflussrechner ausgelegt ist, das Fluid zu analysieren, wobei eine solche Analyse ein Ableiten von Einfallzählraten durch Fitten eines modellierten Spektrums an das gemessene Spektrum umfasst, wobei das modellierte Spektrum die auf ein Einfallsspektrum angewandte Detektorantwortfunktion ist.

**9.** Mehrphasen-Durchflussmesser nach Anspruch 8, wobei der Durchflussrechner ausgelegt ist, die Zählraten für die vom Detektor empfangenen Photonen in Echtzeit abzuleiten.

**10.** Mehrphasen-Durchflussmesser nach Anspruch 8, wobei der Durchflussrechner ausgelegt ist, Phasenanteile für das Fluid innerhalb der Fluidleitung in Echtzeit zu berechnen.

**11.** Mehrphasen-Durchflussmesser nach Anspruch 8, der einen Drucksensor (18) umfasst, wobei der Durchflussrechner ausgelegt ist, eine Förderrate für das Fluid zu bestimmen.

**12.** Mehrphasen-Durchflussmesser nach Anspruch 8, wobei der Emitter eine radioaktive Quelle (72) elektromagnetischer Strahlung umfasst.

**13.** Mehrphasen-Durchflussmesser nach Anspruch 8, wobei der Emitter einen elektrischen Röntgenstrahlengenerator umfasst.

**Revendications**

**1.** Procédé comprenant :

l'émission de rayonnement X et gamma (54) dans un fluide (52) ;
la réception de photons du rayonnement X et gamma transmis par l'intermédiaire du fluide présentant différentes énergies au niveau d'un détecteur (16) ;
la modélisation physique du détecteur par :

la détermination d'un ensemble de fonctions de réponse du cristal monoénergétique basées sur les caractéristiques du détecteur ;
la détermination d'une fonction de réponse du détecteur basée sur l'ensemble de fonctions de réponse du cristal monoénergétique et

l'analyse du fluide basée sur les photons reçus au niveau du détecteur, l'analyse du fluide comportant :

la mesure d'un spectre d'énergie des photons ;
la déduction des taux de comptage d'incident par l'ajustement d'un spectre modélisé au spectre mesuré, le spectre modélisé étant la fonction de réponse du détecteur appliquée à un spectre d'incident.

2. Procédé selon la revendication 1, dans lequel la réception des photons au niveau du détecteur comporte la réception des photons qui sont passés par l'intermédiaire d'un fluide multiphase dans une conduite (12).

3. Procédé selon la revendication 2, comprenant :

le calcul des taux d'atténuation des photons par le fluide multiphase pour lesdits au moins deux niveaux d'énergie ; et
le calcul des fractions de phase du fluide multiphase à l'aide des taux d'atténuation calculés.

4. Procédé selon la revendication 1, dans lequel la déduction des taux de comptage d'incident comporte la déduction des taux de comptage d'incident pour un premier niveau d'énergie des photons du rayonnement X reçus et un second niveau d'énergie des photons du rayonnement gamma reçus.

5. Procédé selon la revendication 4, dans lequel le premier niveau d'énergie des photons du rayonnement X reçus est entre 30 keV et 36 keV.

6. 6. Procédé selon la revendication 4, dans lequel le second niveau d'énergie des photons du rayonnement gamma reçus est entre 79 keV et 81 keV.

7. 7. Procédé selon la revendication 1, dans lequel la réception des photons au niveau du détecteur comporte la réception des photons au niveau d'un scintillateur (80) du détecteur.

8. Débitmètre multiphase comprenant :

une conduite de fluide (12) ;
un émetteur (14) et un détecteur (16) de rayonnement électromagnétique (54) comportant les rayonnements X et les rayonnements gamma, l'émetteur et le détecteur agencés par rapport à la conduite de fluide de façon à permettre au détecteur (16) de recevoir les photons transmis depuis l'émetteur par l'intermédiaire d'un fluide (52) à l'intérieur de la conduite de fluide, le détecteur comportant une chaîne de détection (90) présentant un scintillateur (80), un tube photomultiplicateur (82) et un amplificateur (84);
un analyseur multicanaux (188) couplé au détecteur (16) pour recevoir les signaux électriques depuis l'amplificateur et produire en sortie un spectre d'énergie mesuré des photons reçus par le détecteur (16) ; et
un débitmètre-ordinateur (22) configuré pour modéliser physiquement le détecteur par :

la détermination d'un ensemble de fonctions de réponse du cristal monoénergétique basées sur les caractéristiques du détecteur et
la détermination d'une fonction de réponse du détecteur en fonction de l'ensemble de fonctions de réponse du cristal monoénergétique, dans lequel le débitmètre-ordinateur est configuré pour analyser le fluide, une telle analyse comportant la déduction des taux de comptage d'incident par l'ajustement d'un spectre modélisé au spectre mesuré, le spectre modélisé étant la fonction de réponse du détecteur appliquée à un spectre d'incident.

9. Débitmètre multiphase selon la revendication 8, dans lequel le débitmètre-ordinateur est configuré pour déduire les taux de comptage pour les photons reçus par le détecteur en temps réel.

10. Débitmètre multiphase selon la revendication 8, dans lequel le débitmètre-ordinateur est configuré pour calculer des fractions de phase pour le fluide à l'intérieur de la conduite de fluide en temps réel.

11. Débitmètre multiphase selon la revendication 8, comprenant un capteur de pression (18), dans lequel le débitmètre-ordinateur est conçu pour déterminer un débit pour le fluide.

**12.** Débitmètre multiphase selon la revendication 8, dans lequel l'émetteur comporte une source radioactive (72) du rayonnement électromagnétique.

**13.** Débitmètre multiphase selon la revendication 8, dans lequel l'émetteur comporte un générateur électrique de rayonnement X.

10

14 — EMITTER      PRESSURE SENSOR — 18

12 — FLUID CONDUIT       COMPUTER — 22

16 — DETECTOR      OTHER SENSOR — 20

FIG. 1

22

32

30 — PROCESSOR

46 — INPUT DEVICE

42 — LOCAL MEMORY

44 — I/O INTERFACE

34 — VOLATILE MEMORY

48 — OUTPUT DEVICE

FIG. 2

36 — NON-VOLATILE MEMORY

CODED INSTRUCTIONS — 38

DATA — 40

FIG. 3

FIG. 4

FIG. 5

90

92                    82              84

C  →→→  PMT  →→→  A  →

94      96      98      100

# FIG. 6

110

| DETERMINE CRYSTAL RESPONSE FUNCTION | 112 |
|---|---|
| DETERMINE PMT RESPONSE FUNCTION | 114 |
| DETERMINE AMPLIFIER RESPONSE FUNCTION | 116 |

# FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

210

TRANSMIT RADIATION 212

RECEIVE RADIATION 214

MEASURE SPECTRUM 216

INFER VARIABLES OF PHYSICAL MODEL 218

CHARACTERIZE FLUID 220

CALIBRATE DETECTOR 222

FIG. 22

230

EMIT RADIATION THROUGH MULTIPHASE FLUID 232

RECEIVE RADIATION 234

TRANSFORM RADIATION TO ELECTRICAL SIGNALS 236

DETERMINE ENERGY SPECTRUM 238

DECONVOLVE ENERGY SPECTRUM 240

CALCULATE ATTENUATION COEFFICIENTS 242

DETERMINE PHASE FRACTIONS 244

FIG. 23

FIG. 24

FIG. 25

× SPECTRUM —— SMEARED SPECTRUM ---- OBSERVED SPECTRUM
—— 26.73 ·--·- 59.51 -·—·- 103.61

## FIG. 26

× SPECTRUM —— SMEARED SPECTRUM ---- OBSERVED SPECTRUM
-·—·- 31 —— 36 ·—·- 511 ·—··- 662 ---- 1274

## FIG. 27

250

252

RECEIVE PHOTONS

254

MEASURE SPECTRUM

256

DERIVE SPECTRAL
COMPONENTS

258

MEASURE COUNT
RATES

260

CALCULATE
ATTENUATION

262

CALCULATE PHASE
FRACTIONS

FIG. 28

270

272

TRANSMIT RADIATION

274

RECEIVE RADIATION
AT SCINTILLATOR

276

RECEIVE LIGHT
AT PMT

278

CONVERT LIGHT TO
ELECTRICAL SIGNALS

280

MEASURE ENERGY
SPECTRUM

282

OPTIMIZE VARIABLES
OF RESPONSE MODEL

284

CALCULATE
ATTENUATION

286

CALCULATE PHASE
PROPORTIONS

FIG. 29

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2574919 A1
- EP 1970702 A1
- WO 0250522 A1
- WO 2009036337 A2
- WO 0125762 A1
- WO 2009058964 A1

**Non-patent literature cited in the description**

- **LAVIGNE et al.** Extraordinary improvement in scintillation detectors via post-processing with ASEDRA-solution to a 50-year-old problem. *SPIE,* 01 January 2008
- **MENG ; D RAMSDEN L J.** An inter-comparison of three spectral-deconvoultion algorithms for gammy-ray spectroscopy. *IEEE Transactions on Nuclear Science,* 01 August 2000, vol. 47 (4), 1329-1336
- An analytic model for the response of a CZT detector in diagnostic energy dispersive x-ray spectroscopy. **LECLAIR ROBERT et al.** Medical Physics. AIP, 24 April 2006, vol. 33, 1329-1337
- Calibration and characterization of semiconductor X-ray detectors with synchrotron radiation. **KRUMREY, M et al.** Nuclear Instruments & Methods in Physics Research, Section A: Accelerators, Spectrometers, Detectors, and Associated Equipment. Elsevier BV, 30 November 2006, vol. 568, 364-368